# EUROPEAN PATENT SPECIFICATION

(11) **EP 0 656 422 B1**
(45) Date of publication and mention of the grant of the patent: **26.04.2000**
(21) Application number: 94307991.3
(22) Date of filing: 31.10.1994
(51) Int. Cl.: C12Q 1/48, G01N 33/94, G01N 33/532

(54) **Chloramphenicol acetyl transferase (CAT) assay**
Chloramphenicol Acetyl Transferase (CAT) Nachweis
Essai de chloramphenicol-acetyl-transférase (CAT)

(30) Priority: 05.11.1993 EP 93308860
(43) Date of publication of application: 07.06.1995
(73) Proprietor: Amersham Pharmacia Biotech UK Limited, Little Chalfont, Buckinghamshire HP7 9NA (GB)
(72) Inventor: Brophy, Gerard Philip, Llandaff, Cardiff CF5 2DN (GB); Cummins, William Jonathan, Tring, Hertfordshire HP23 4JE (GB); Mundy, Christopher Robert, Cambridge CB3 OEL (GB)
(74) Representative: Pennant, Pyers

(56) References cited:
- EP-A- 0 387 875
- WO-A-89/03042
- US-A- 4 489 156
- US-A- 4 489 157
- US-A- 4 568 649
- DATABASE WPI Week 9127, Derwent Publications Ltd., London, GB; AN 91-198111 & JP-A-3 123 864 (TOSOH CORP) 27 May 1991
- CHEMICAL ABSTRACTS, vol. 90, no. 1, 1 January 1979, Columbus, Ohio, US; abstract no. 90:106x, A.L.SMITH ET AL. 'Improved enzymic assay of chloramphenicol' page 5 ;column 2 ; & CLINICAL CHEMISTRY., vol.24, no.9, 1978, WINSTON US pages 1452 - 1457

## Description

### INTRODUCTION

Chloramphenicol Acetyl Transferase (CAT) is the most commonly used reporter gene in molecular biology. The enzyme catalyses the reaction of chloramphenicol and acetyl coenzyme A to produce acetylated chloramphenicol. The enzyme has no eukaryotic counterpart and therefore is a useful measure of transfection efficiency and translational activity in cultured cells. Typically the gene, contained on a plasmid, is transfected into the cell of interest and, after an appropriate incubation period, CAT activity is measured.

CAT has a number of advantages for use as a reporter gene:
- The enzyme activity is readily distinguishable from that of endogenous cellular proteins present in the cell prior to transfection.
- There is no interference or competition from other enzymatic activities in the cells.
- CAT is a relatively stable enzyme exhibiting a degree of thermotolerance and a lack of inhibition by cellular components.

### PRESENT METHODS OF CAT ACTIVITY DETERMINATION

### ¹⁴C Chloramphenicol TLC

This is the most common method of assaying for CAT activity. The cell extract containing CAT is incubated with ¹⁴C chloramphenicol and cold acetyl coA. After incubation the reactants are extracted into an organic solvent and dried down. The reactants are resuspended in a small volume and spotted on Thin Layer Chromatography (TLC) plates which are developed to separate chloramphenicol from its acetylated derivatives. The plates are then exposed to autoradiographic film to locate the radioactive spots. These can then be scraped off, extracted and scintillation counted to give a measure of acetylation activity. Alternatively if a radioactive imaging system, such as a phosphorimager, is available the plates can be exposed and directly quantified by this means.

The disadvantages of this method are as follows:
- The assay is cumbersome and time consuming.
- The assay involves TLC.
- Organic solvents are used.
- Quantification is difficult.
- Sensitivity is limited.
- It is difficult to assay large numbers of samples.

### Labelled acetyl coA

A variation on the above assay uses a radioactive acetyl group on the coA and cold chloramphenicol. After the reaction the mix is extracted with organic solvent or overlayed with water immiscible scintillation fluid. Acetylated forms of chloramphenicol diffuse into the organic phase and can be counted.

### Disadvantages:

- The assay is difficult to perform and often yields unsatisfactory results due to phase flipping problems.

### Fluorescent chloramphenicol

A chloramphenicol with a fluorescent molecule attached is commercially available. This assay format is carried out by adding this fluorescent chloramphenicol and acetyl coA to the cell extract. The chloramphenicol and its reaction products are separated by TLC and visualised by UV illumination. The spots can be scraped off and quantified in a fluorimeter.

### Disadvantages:

- The assay is cumbersome and time consuming.
- The assay requires a TLC separation step.
- Sensitivity is low.
- A fluorimeter may not be easily accessible in every laboratory.

### CAT ELISA

A CAT ELISA kit is commercially available. In this assay format cell extract is pipetted into MTP wells which contain bound CAT antibody. The wells are washed and incubated with anti-CAT digoxygenin. Again the wells are washed and incubated with anti-digoxygenin peroxidase. The wells are washed and incubated with a peroxidase substrate yielding a colourimetric endpoint which can be quantified on an MTP reader.

### Disadvantages:

- CAT presence not activity is measured.
- The assay is relatively time consuming due to the many washing steps.
- Sensitivity is low.

### Prior Art

EP-A-0 387 875 discloses an assay for a glycosyl transferase which comprises incubating a sample containing the enzymes with an oligosaccharide acceptor coupled to a protein, a donor sugar, and an antibody specific for the said protein. The amount of sugar found attached to the oligosaccharide-antibody complex is a measure of the enzyme activity.

US-A-4,489,157 and US-A-4,489,156 both provide chloramphenicol derivatives for use in the preparing of antigen conjugates for the production of antibodies specifically for chloramphenicol.

### THE INVENTION

This invention provides an improved method of assaying for CAT, based on the use of chloramphenicol analogues, some of which are also claimed herein as new compounds.

In one aspect the invention provides a method of assaying for CAT in a fluid, which method comprises incubating in the presence of a sample of the fluid, a chloramphenicol analogue which comprises a member of a specific binding pair, a radiolabelled acyl coenzyme A analogue, and a third reagent which comprises the other member of the specific binding pair, whereby there is formed a complex
(third reagent) - (chloramphenicol analogue) - (acyl group)
to an extent related to the CAT activity of the fluid sample.

A chloramphenicol analogue is a compound, related to chloramphenicol, which is capable of reacting with acetyl coenzyme A, in a reaction catalysed by CAT, to give the 3-acetyl derivative. The analogue is a modified chloramphenicol derivative, which may for example have lost its dichloroacetyl function.An analogue may be chemically identical to the originating compound; for example, a radiolabelled compound is regarded herein as being an analogue of the unlabelled compound.

Specific binding pairs are well known in biochemistry. They include, but are not limited to: antigen/antibody; hapten/antibody; biotin/avidin; biotin/streptavidin; single stranded DNA/complementary DNA.

The chloramphenicol analogue comprises a member of a specific binding pair, and may be a conjugate of chloramphenicol with the member of the specific binding pair, the conjugate having the structure shown 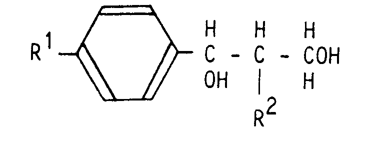
where R¹ is -NO₂ or -X-Z,
R² is -NHCOR³ or -X-Z, provided that at least one of R¹ and R² is -X-Z,
R³ is CHCl₂ or C₁-C₆ alkyl,
Z is a member of a specific binding pair, e.g. a hapten, and
X is a spacer arm of sufficient length to enable the hapten or other member of a specific binding pair to bind correctly with its specific binding partner.

When R¹ is NO₂ and R² is NHCOCHCl₂, the compound is chloramphenicol. The two asymmetric carbon atoms generate four possible stereoisomers, of which only the D-threo isomer has significant biological activity. Preferred chloramphenicol analogues are based on this stereoisomer.

Z may be a hapten such as biotin, fluorescein or digoxygenin. The spacer arm X may typically be from 2 to 25 carbon atoms, preferably 6 carbon atoms in length. Certain of these chloramphenicol analogues are new compounds, and are included as such in this invention. Among these are compounds having the formula 1 above in which
either R¹ is NO₂ and R² is X-biotin,
or R¹ is X-biotin and R² is NHCOCH₃,
and X is a spacer arm of sufficient length to enable the biotin moiety to bind correctly with its specific binding partner. Preferably X contains 6 carbon atoms, so that the compound is either (+)-D-threo-6-biotinylamino-N[(2-hydroxy-1-hydroxymethyl)-2-(4-nitrophenyl) ethyl]-hexamide; or 6-biotinylamino-N[4-(N-acetyl-1-(2-amino-1, 3-propandiol))phenyl] hexamide.

An acyl coenzyme A analogue is a compound, related to acetyl coenzyme A, which is capable of undergoing a reaction with chloramphenicol, catalysed by CAT, resulting in the production of a 3-acyl derivative of chloramphenicol. The acyl coenzyme A analogue may be acetyl coenzyme A, radiolabelled with e.g. tritium or carbon-14. Or the acyl coenzyme A analogue may be an acyl coenzyme A, in which the acyl is e.g. propionyl or butyryl. The acyl group of the acyl coenzyme A analogue is radiolabelled. The nature of the radiolabel is not material to the invention.

The method of the invention involves the use of a third reagent which is or comprises a member of a specific binding pair; the other member of this specific binding pair is comprised in the chloramphenicol analogue. For example, when the biotinylated chloramphenicol analogue is used, the third reagent may be avidin or streptavidin.

The chloramphenicol analogue, the acyl coenzyme A analogue and the third reagent are incubated, in the presence of a sample of the fluid under test, in order to form a complex (third reagent) - (chloramphenicol analogue) - (acyl group).

In this complex, the acyl group is radiolabelled. In order to facilitate separation of the complex from unreacted label, the third reagent may be bound to a solid support, for example to a wall of an assay vessel or to solid particles suspended in the assay medium. The assay reagents may be incubated all together, or alternatively the chloramphenicol analogue may be pre-reacted with the third reagent.

There follow examples of preferred assay formats according to the invention. These involve the use of chloramphenicol-hapten conjugates as the chloramphenicol analogue. The hapten is used for separation/attachment purposes and the radiolabelled acyl group provides the means of detection.

### A. SPA-CAT Assay

SPA, the Scintillation Proximity Assay is covered by U.S. Patent No. 4,568,649, European Patent No. 0154734 and Japanese Patent Application No. 84/52452.

In this assay format a haptenated chloramphenicol analogue (the hapten is preferably biotin) is incubated with tritiated (³H) acetyl coenzyme A. Suitably coated SPA beads (e.g. streptavidin or specific antibodies) are added and bind to the acetylated, haptenated chloramphenicol analogue produced giving rise to a scintillation signal which is a measure of enzyme activity.

The assay is quick and easy to use. Results are possible in one hour after extract production. No separation steps are required and the entire assay procedure can be carried out in one tube.

In addition the assay is very sensitive (at least fifteen fold more sensitive than ¹⁴C chloramphenicol TLC assays).

### B. CAT assay with coated beads.

In this assay format tritiated, acetylated, haptenated chloramphenicol analogue is produced as before in a microcentrifuge tube. Suitably coated beads are added which bind to the haptenated molecule. The beads are centrifuged to form a pellet and the supernatant, which contains the unreacted tritiated acetyl coenzyme A, is removed. The pellet is washed and scintillation fluid added. The scintillation signal produced is a measure of CAT activity.

The assay, although not homogeneous, requires only a single separation step and can be carried out in one tube. It is therefore quicker and easier and more sensitive than existing CAT assays.

Reference is directed to the accompanying drawings in which:-
Figure 1 is a diagram showing the synthesis of (+)-D-threo-6-biotinylamino-N[(2-hydroxy-1-hydroxymethyl)-2-(4-nitrophenyl)ethyl]-hexanamide (Compound 1).
Figure 2 is a mass spectrometry trace of Compound 1.
Figure 3 is a diagram showing the synthesis of 6-biotinylamino-N[4-(N-acetyl-1-(2-amino-1,3-propandiol))phenyl]hexamide (Compound 2).

The following examples illustrate the invention.

### General Details

Biotinamidocaproic acid N-hydroxysuccinimide ester was obtained from Sigma. D-threo-2-amino-1-(4-nitrophenyl)-1,3-propandiol was obtained from Aldrich or produced by base hydrolysis of chloramphenicol. Triethylamine was predistilled from sodium hydroxide. Anhydrous solvents refer to Aldrich Sure Seal bottled material. Other solvents and reagents are available from several commercial sources.

¹H NMRs were obtained on a Jeol 270 MHz machine using d₄ MeOH as solvent.

TLCs were run on Kieselgel 60 F₂₅₄ aluminium backed TLC plates using the solvent system quoted.

### EXAMPLE 1

### Synthesis of (+)-D-threo-6-biotinylamino-N[2-hydroxy-1-(hydroxymethyl)-2-(4-nitrophenyl)ethyl]-hexamide. (Compound 1)

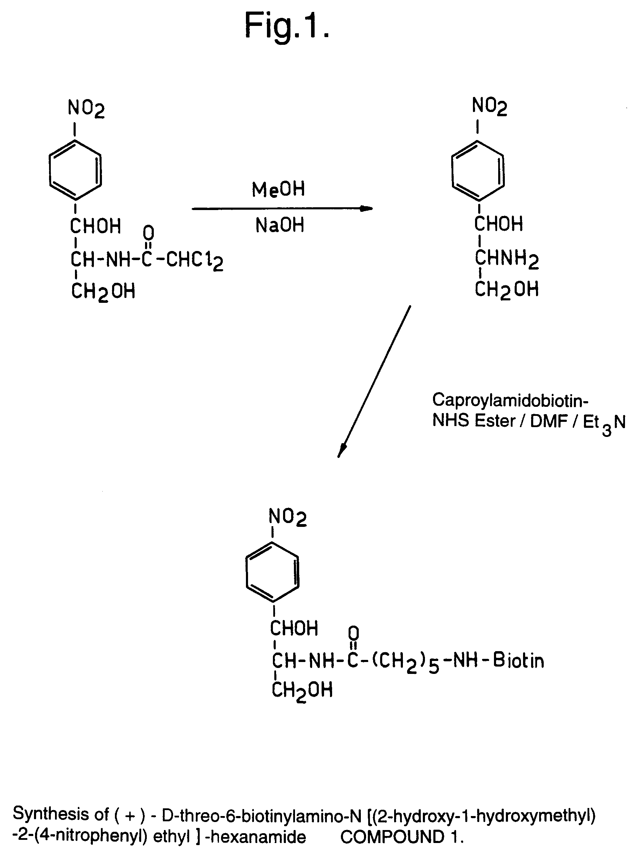

The synthetic reaction sequence is shown in Figure 1.

In a clean, round-bottom flask biotinamidocaproic acid N-hydroxysuccinimide ester (230 mg 0.51 mmoles) was dissolved in anhydrous dimethylformamide (4 ml). To this was added D-threo-2-amino-1-(4-nitrophenyl)-1,3-propandiol (110 mg 0.52 mmoles) and triethylamine (50 µl). The flask was then stoppered and the contents stirred overnight at room temperature.

Product formation was verified by pipetting 0.5 µl of the reaction onto a TLC plate and developing in chloroform/methanol 9:1. The product has an R_{f} of 0.2.

The product was purified using a preparative scale reverse phase HPLC column (Hamilton PRP-1 preparative column 21:4 mm id x 250 mm). An acetonitrile/water gradient elution was used at a flow rate of 8 ml/min.

### Gradient profile

| Time (min) | % acetonitrile |
|---|---|
| 0 | 10 |
| 40 | 50 |
| 45 | 100 |
| 50 | 10 |

The product peak's retention time was approximately 30 mins as measured by UV absorption at 254 nm. The solvent from the product fraction was rotary evaporated off and the product was resuspended in ethyl acetate (5 ml). The white solid was filtered on a sintered glass funnel and then air dried. The solid was placed under high vacuum for one hour to remove the last traces of solvent. Yield 200 mg (71%). The melting point was determined as 173°C → 175°C. 270 MHz ¹H NMR d₄MeOH δ: 1.1→1.8 (m, 12H), 2.1 (t, 2H), 2.7 (d, 1H), 2.9 (m, 1H), 3.1 (m, 2H), 3.2 (m, 1H), 3.5 (m, 1H), 3.7 (m, 1H), 4.1 (m, 1H), 4.3 (m, 1H), 4.5 (m, 1H), 5.1 (d, 1H), 7.6 (d, 2H), 8.2 (d, 2H).

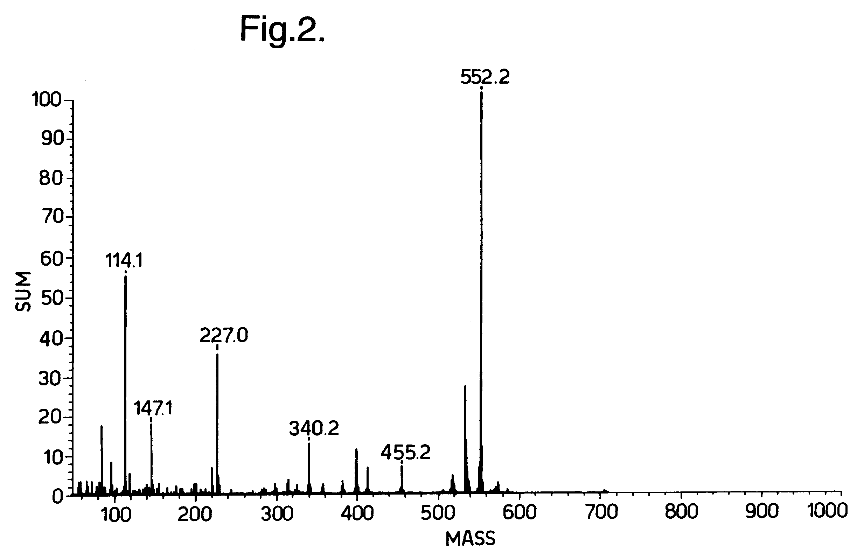

The product was analysed by mass spectrometry. A trace is provided in Figure 2.

A kinetic analysis of CAT and this biotinylated chloramphenicol analogue was carried out to characterise this new substrate. This work determined that the enzyme's Km for chloramphenicol was 11.0 µM and for the biotinylated chloramphenicol analogue was 9.5 µM. The kcats were 97 s⁻¹ and 93 s⁻¹ respectively. These data indicate that the biotinylated chloramphenicol analogue is a suitable substrate for CAT and that the reaction rates of the enzyme are similar with both substrates.

### EXAMPLE 2

### Standard curve of Chloramphenicol Acetyl Transferase (CAT) activity.

In this assay the biotinylated chloramphenicol analogue of Example 1 is acetylated by CAT using [³H] acetyl coenzyme A as the acyl donor. The tritiated acetylated biotinylated chloramphenicol analogue binds to Scintillation Proximity Assay (SPA) beads and gives rise to a scintillation signal which can be related to enzyme activity.

### Materials and Methods

CAT enzyme obtained from Professor W. V. Shaw, Biochemistry Department, Leicester University, was diluted to concentrations of 0.02, 0.04, 0.06, 0.08 and 0.1 units of enzyme with 0.1 M Tris/HCl (pH 7.8) in total volume of 40 µl. One unit of enzyme is the amount which catalyses the acetylation of one nanomole of chloramphenicol in one minute at 37°C. Duplicate samples were assayed. To each assay tube was added 10 µl of a master mix which contained:

| | |
|---|---|
| biotinylated chloramphenicol analogue (0.1 mmolar) | 1 µl |
| [³H] acetyl coenzyme A (Amersham TRK688) | 2 µl (0.5 µCi) |
| water | 1 µl |
| Tris/HCl, pH 7.8, 1 molar | 6 µl. |

The reaction mixture was mixed and incubated at 37°C for 30 minutes.

After incubation 0.5 mg of streptavidin coated SPA beads (Amersham) were added in 0.75 ml PBS. The reaction tubes were placed in scintillation vials and counted.

The results are tabulated below.

| CAT (units) | cpm |
|---|---|
| 0 | 181 |
| | 197 |
| 0.02 | 11304 |
| | 11485 |
| 0.04 | 24026 |
| | 23873 |
| 0.06 | 34921 |
| | 34957 |
| 0.08 | 46748 |
| | 45050 |
| 0.1 | 50846 |
| | 52762 |

As can be seen the increase in CAT activity is proportional to the increase in cpm.

### EXAMPLE 3

### Synthesis of 6-biotinylamino-N[4-(N-acetyl-1-(2-amino-1,3-propandiol))phenyl]hexamide (Compound 2).

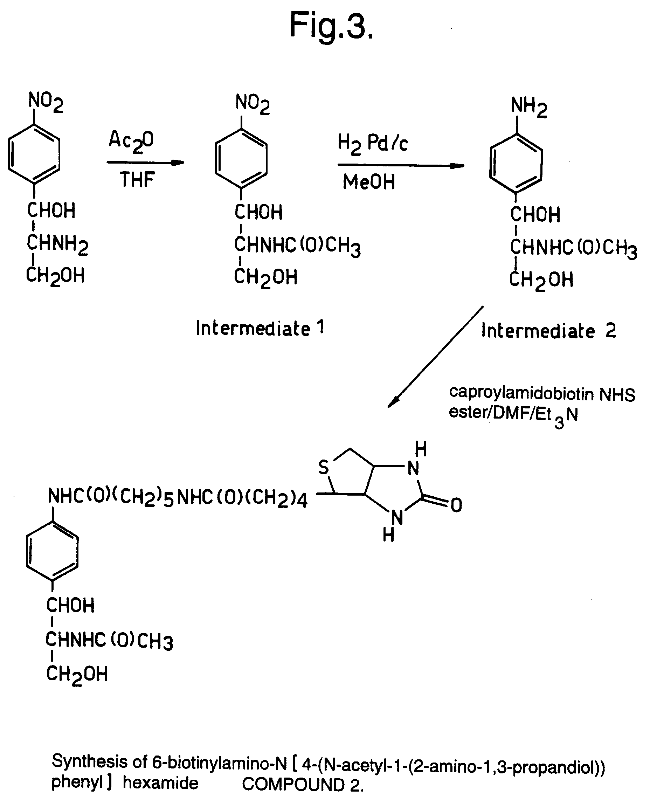

The synthetic reaction sequence is shown in Figure 3.

The synthetic details for each intermediate compound in the synthetic sequence and for the final synthetic step are set out below.

### N-[2-hydroxy-1-(hydroxymethyl)-2-(4-nitrophenyl)ethyl] acetamide. Intermediate 1

In a clean, round bottom flask D-threo-2-amino-1-(4-nitrophenyl)-1,3-propandiol (1.43 g, 6.75 mmoles) was suspended in anhydrous tetrahydrofuran (30 ml). To this was added acetic anhydride (0.8 ml, 8.44 mmoles). The flask was stoppered and the contents stirred at room temperature for one hour.

Product formation was verified by spotting a small aliquot of the reaction mixture onto a TLC plate and developing in ethyl acetate/methanol 9:1. The product has an R_{f} of 0.33 and is negative to a ninhydrin spray.

The tetrahydrofuran was removed on a rotary evaporator and the resulting gummy residue subjected to a silica flash column to purify the product. The eluant for the column was ethyl acetate/methanol 9:1. The product fractions were pooled and the solvent removed on a rotary evaporator. The resulting foam was placed under high vacuum for one hour to remove the last trace of solvent. The yield was 1.28 g (78%). 270 MHz ¹H NMR d₄MeOH δ: 1.8 (s, 3H), 3.5 (m, 1H), 3.7 (m, 1H), 4.1 (m, 1H), 5.1 (brs, 1H), 7.6 (d, 2H), 8.2 (d, 2H).

### N-[2-hydroxy-1-(hydroxymethyl)-2-(4-aminophenyl)ethyl] acetamide. Intermediate 2

In a clean, round bottom flask N-[2-hydroxy-1-(hydroxymethyl)-2-(4-nitrophenyl)ethyl] acetamide (1.27 g 5 mmoles) was dissolved in methanol (50 ml). The flask was then fitted with a suitable adaptor and coupled to an hydrogenation apparatus. The atmosphere in the flask was then replaced with nitrogen. The adaptor was then removed and 10% palladium on carbon catalyst (466 mg) was added to the flask. The adaptor was replaced and the hydrogenation apparatus manipulated to replace the atmosphere in the flask with hydrogen. The flask contents were then stirred at room temperature under an atmosphere of hydrogen overnight. The hydrogenation apparatus was manipulated to replace the atmosphere in the flask with nitrogen. The flask was then disconnected from the hydrogenation apparatus.

The palladium on carbon catalyst was removed by filtering through a hyflo filter pad. The solvent from the product was rotary evaporated off to give a gum. The gum was placed under high vacuum for one hour to remove the last traces of solvent. The yield of product was 1.05 g (94%). 270 MHz ¹H NMR d₄MeOH δ: 1.9 (s, 3H), 3.4 (m, 1H), 3.6 (m, 1H), 4.0 (m, 1H), 4.7 (d, 1H), 6.7 (d, 2H), 7.1 (d, 2H).

The Intermediate 2 product was used in the next synthetic step without any purification.

### 6-biotinylamino-N[4-(N acetyl-1-(2 amino-1,3-propandiol))phenyl]hexamide

Biotinamidocaproic acid N-hydroxysuccinimide ester (100 mg 0.22 mmoles) was weighed out into a clean, round bottomed flask. A solution of N-[2-hydroxy-1-(hydroxymethyl)-2-(4-aminophenyl)ethyl] acetamide (108 mg, 0.51 mmoles) in anhydrous dimethylformamide (2 ml) was added to the flask. Triethylamine (25 µl) was added to the flask which was then stoppered. The reaction mixture was stirred at room temperature for a total of 80 hours and at 80°C for a total of 6 hours.

The major product was then purified using a preparative scale reverse phase HPLC column (Hamilton PRP-1 preparative column 21.4 mm id x 250 mm). An acetonitrile/ammonia solution (2ml of 0.880 NH₃/litre of water ) gradient elution was used at a flow rate of 8 ml/min.

### Gradient profile

| Time (min) | % acetonitrile |
|---|---|
| 0 | 0 |
| 45 | 40 |
| 50 | 100 |
| 55 | 0 |

The product peak retention time was approximately 35 min as measured by UV absorption at 254 mm. The solvent from the product fraction was rotary evaporated off to give an off white solid. the yield was 24 mgs (20%). 270 MHz ¹H NMR d₄MeOH δ: 1.2 → 1.7 (m, 12H), 1.9 (s, 3H), 2.1 (t, 2H), 2.3 (t, 2H), 2.6 (d, 1H), 2.8 (m, 1H), 3.1 (m, 3H), 3.4 (m, 1H), 3.5 (m, 1H), 4.0 (m, 1H), 4.3 (m, 1H), 4.4 (m, 1H), 7.2 (d, 2H), 7.4 (d, 2H).

### EXAMPLE 4

### 6-biotinylamino-N[4-(N-acetyl-1-(2-amino-1,3-propandiol))phenyl]hexamide, (Compound 2) is a substrate of CAT.

Biotinylated chloramphenicol analogue (compound 2) was dissolved to a concentration of 2 mmolar in 50% methanol. ¹⁴C acetyl coenzyme A (50 µCi/ml, 58 mCi/mole) was obtained commercially.

In a microcentrifuge tube were mixed Tris/HCl (1M, pH 7.8) 7.5 µl, biotinylated chloramphenicol analogue (2 mM) 80 µl, CAT enzyme 3.75 units and ¹⁴C acetyl coenzyme A 2 µl (0.1 µCi). The mix was incubated at 37°C for 90 minutes. A control reaction which contained no enzyme was performed in parallel. 20 µl of each reaction was spotted on to a cellulose TLC plate which was developed in 95/5 chloroform methanol. The TLC plate was exposed to autoradiographic film.

The lane corresponding to the reaction to which enzyme was added contains a spot which migrated under the influence of the solvent. This band is acetylated, biotinylated chloramphenicol analogue. No corresponding band is visible in the no enzyme control.

This experiment proves that 6-biotinylamino-N[4-(N acetyl-1-(2-amino-1,3-propandiol))phenyl]hexamide is acetylated with ¹⁴C acetyl coenzyme A by CAT.

## Claims

1. A method of assaying for chloramphenicol acetyl transferase (CAT) in a fluid, which method comprises incubating in the presence of a sample of the fluid, a chloramphenicol analogue which comprises a member of a specific binding pair, a radiolabelled acyl coenzyme A analogue, and a third reagent which comprises the other member of the specific binding pair, whereby there is formed a complex
(third reagent) - (chloramphenicol analogue) - (acylgroup)to an extent related to the CAT activity of the fluid sample.

2. A method as claimed in claim 1, wherein the third reagent is bound to a solid support.

3. A method as claimed in claim 1 or claim 2, wherein the acyl coenzyme A analogue is radioactively labelled acetyl coenzyme A.

4. A method as claimed in any one of claims 1 to 3, wherein the acyl coenzyme A analogue is acetyl coenzyme A labelled with tritium, and the third reagent is immobilised on a solid support.

5. A method as claimed in any one of claims 1 to 4, wherein the solid support is a scintillation proximity assay support.

6. A method as claimed in any one of claims 1 to 5, wherein the chloramphenicol analogue is a biotinylated chloramphenicol analogue, and the third reagent is streptavidin or avidin.

7. A method as claimed in any one of claims 1 to 6, wherein the chloramphenicol analogue is (+)-D-threo-6-biotinylamino-N[(2-hydroxy-1-hydroxymethyl)-2-(4-nitrophenyl) ethyl]-hexamide.

8. 6-Biotinylamino-N[4-(N-acetyl-1-(2-amino-1,3-propandiol))phenyl]hexamide.

## Patentansprüche

1. Verfahren zur Bestimmung von Chloramphenicolacetyltransferase (CAT) in einem Fluid, wobei das Verfahren Inkubieren eines Chloramphenicolanalogen, das ein Mitglied eines spezifischen Bindungspaars umfaßt, eines radioaktiv markierten Acylcoenzym-A-analogen und eines dritten Reagenzes, das das andere Mitglied des spezifischen Bindungspaares umfaßt, in Gegenwart einer Probe des Fluids umfaßt, wodurch ein Komplex
(drittes Reagenz)-(Chloramphenicolanaloges)-(Acylgruppe) in einem im Verhältnis zur CAT-Aktivität der Fluid-Probe stehenden Ausmaß gebildet wird.

2. Verfahren nach Anspruch 1, wobei das dritte Reagenz an einen festen Träger gebunden ist.

3. Verfahren nach Anspruch 1 oder Anspruch 2, wobei das Acylcoenzym-A-analoge radioaktiv markiertes Acetylcoenzym A ist.

4. Verfahren nach einem der Ansprüche 1 bis 3, wobei das Acylcoenzym-A-analoge Acetylcoenzym A, markiert mit Tritium, ist und das dritte Reagenz auf einem festen Träger immobilisiert ist.

5. Verfahren nach einem der Ansprüche 1 bis 4, wobei der feste Träger ein Szintillation-Proximity-Assay-Träger ist.

6. Verfahren nach einem der Ansprüche 1 bis 5, wobei das Chloramphenicolanaloge ein biotinyliertes Chloramphenicolanaloges ist und das dritte Reagenz Streptavidin oder Avidin ist.

7. Verfahren nach einem der Ansprüche 1 bis 6, wobei das Chloramphenicolanaloge (+)-D-Threo-6-biotinylamino-N[(2-hydroxy-1-hydroxymethyl)-2-(4-nitrophenyl)ethyl]hexamid ist.

8. 6-Biotinylamino-N[4-(N-acetyl-1-(2-amino-1,3-propandiol))phenyl]hexamid.

## Revendications

1. Procédé de dosage de la chloramphénicol acétyl-transférase (CAT) dans un liquide, lequel procédé comprend l'incubation, en présence d'un échantillon du liquide, d'un analogue de chloramphénicol constituant un membre d'une paire de liaison spécifique, d'un analogue d'acyl-coenzyme A radiomarqué et d'un troisième réactif qui constitue l'autre membre de la paire de liaison spécifique, ce par quoi il se forme un complexe (troisième réactif) - (analogue de chloramphénicol) - (groupe acyle) à un degré qui est en relation avec l'activité CAT de l'échantillon de liquide.

2. Procédé selon la revendication 1, dans lequel le troisième réactif est fixé à un support solide.

3. Procédé selon la revendication 1 ou 2, dans lequel l'analogue d'acyl-coenzyme A est l'acétyl-coenzyme A radiomarquée.

4. Procédé selon l'une quelconque des revendications 1 à 3, dans lequel l'analogue d'acyl-coenzyme A est l'acétyl-coenzyme A marquée au tritium, et le troisième réactif est immobilisé sur un support solide.

5. Procédé selon l'une quelconque des revendications 1 à 4, dans lequel le support solide est un support d'essai de proximité en scintillation.

6. Procédé selon l'une quelconque des revendications 1 à 5, dans lequel l'analogue de chloramphénicol est un analogue de chloramphénicol biotinylé, et le troisième réactif est la streptavidine ou l'avidine.

7. Procédé selon l'une quelconque des revendications 1 à 6, dans lequel l'analogue de chloramphénicol est le (+)-D-*threo*-6-biotinylamino-N[(2-hydroxy-1-hydroxyméthyl)-2-(4-nitrophényl)éthyl]hexamide.

8. 6-biotinylamino-N[4-(N-acétyl-1-(2-amino-1,3-propanediol))phényl]hexamide.
